# EUROPEAN PATENT APPLICATION

(11) **EP 3 320 938 A1**
(43) Date of publication of application: **16.05.2018**
(21) Application number: 17200676.9
(22) Date of filing: 08.11.2017
(51) Int. Cl.: A61M 15/00

(54) **INHALER DEVICE WITH IMPROVED OUTER BODY**

(30) Priority: 09.11.2016 TR 201616027; 22.12.2016 TR 201619235; 30.12.2016 TR 201620257
(71) Applicant: Arven Ilac Sanayi Ve Ticaret A.S., Istanbul 34460 (TR)
(72) Inventor: TOKSÖZ, Ahmet, 34460 Istanbul (TR); TOKSÖZ, Zafer, 34460 Istanbul (TR)
(74) Representative: Sevinç, Erkan

(57) **Abstract**

The present invention relates to an outer body (8) for a dry powder inhaler device (1), wherein said outer body (8) comprises at least one rib (8.1) at the inner surface thereof.

## Description

### Technical Field

The present invention relates to a device providing the administration of dry powder inhalation medicament. The present invention particularly relates to advancements in the outer body structure of dry powder inhaler devices.

### Prior Art

Despite the advances in diagnosis and treatment in recent years, diseases such as asthma, bronchitis and COPD (Chronic Obstructive Pulmonary Disease) decrease the quality of life of humans. The administration of medicaments by means of inhalers is recommended for optimizing the treatment of these types of diseases. The treatment by means of inhalers is the most preferred treatment type and is expected to be the first option in the future. The most important advantage of using medicaments by means of inhalation is to provide a more efficient treatment by using less medicaments, to administer medicaments in a higher concentration the respiratory tract and in particular to decrease the systemic side effects of medicaments. Despite the presence of very efficient treatments for respiratory tract diseases, the most significant reasons of failing to properly control patients are stated as noncompliance with the treatments recommended by the physicians and incoordination due to the improper use of inhalers.

In recent years, various inhalation devices have been developed for administering inhalation medicaments. These devices are basically categorized into two types: metered-dose inhalers and dry powder inhalers. These types of devices structurally comprise basic components such as mechanism, counter, body, lid, lock, etc. In addition, powder inhalation medicaments are preserved in carriers such as blister, capsule, etc. Blisters are composed of two basic components, namely the main layer with cavities for carrying the medicament and the strippable lid layer.

The type of dry powder inhaler devices with blisters comprises a mechanism that moves the blister forward and provides the sequential use of cavities in the blister. The patient uses an arm or a trigger to move said mechanism. This type of devices has main sections such as advancement mechanism, counter and lock and mechanism that form the same. Said sections and mechanisms are positioned and protected in an outer body. In the state of the art inhaler devices, various body embodiments are used. In patent applications no. EP2239001B1 and EP2239002B1, a two-piece body assembly is disclosed. Two pieces of said body engage with each other by means of opposite teeth and housings, thus forming a closed receptacle/casing assembly. Moreover, various pins and housings are provided on the inner surface of said body pieces. An inner body and gears and components forming the mechanism are connected to said pins and housings. Said outer body is designed to be very thick so as to protect the mechanism and components disposed therein. Thus, the surfaces of the body resist against outer factors such as impact, pressure, etc. However, this causes a bulky device and inconveniences in terms of carrying. Moreover, this situation causes increase in materials used.

Consequently, in the relevant technical field, a novelty providing operation with the desired precision and advantage in volume and preventing waste of materials is required in order to eliminate said problems in the inhaler devices.

### Objects and Brief Description of the Invention

The present invention relates to an improved inhaler device and outer body used in dry powder inhalation operation, that eliminate all the above-mentioned problems and that provide advantages to the relevant technical field.

With respect to the state of the art, the principal aim of the present invention is to provide a dry powder inhaler device that operates with the desired precision, that enables the blisters to be stripped and used and that realizes this operation in an error-free manner with respect to the precedent devices.

Another aim of the present invention is to provide an inhaler device that is strengthened by means of rib or ribs and that better resists against outer factors such impact and adverse effects.

Yet another aim of the present invention is to provide an inhaler device that has thinner body walls, hence a smaller volume, by means of the ribs.

Yet another aim of the present invention is to provide an inhaler device that has thinner body walls by means of the ribs, which can be produced with reduced amount of materials.

In order to attain all the aims that are described above and that may arise from the below detailed description, a novelty in the outer body of the dry powder inhaler device is realized.

In a preferred embodiment of the present invention, said novelty is realized by means of at least one rib provided on the inner surface of said outer body.

In a preferred embodiment of the present invention, said rib is shaped in one or more of the linear, S and Z forms. The rib is preferably linear.

In a preferred embodiment of the present invention, two or more ribs are provided.

In a preferred embodiment of the present invention, said ribs are positioned parallel to each other.

In a preferred embodiment of the present invention, said ribs are positioned so as intersect each other.

### Brief Description of Figures

Figure 1 shows the perspective view of a representative embodiment of the inhaler device of the present invention.
Figure 2 shows the representative perspective view of the inhaler device of the present invention in the disassembled state.
Figure 3 shows the representative perspective view of the inhaler device of the present invention in the disassembled state.
Figure 4 shows the detailed perspective view of the outer body disposed in the inhaler device of the present invention.
Figure 5 shows the view of a representative embodiment of the blister disposed in the inhaler device of the present invention.
Figure 6 shows the detailed perspective view of the locking clip disposed in the inhaler device of the present invention.

### Reference Numerals used in Figures

1. Inhaler device
2. Clip
2.1 Clip spring
3. Clip first end retaining tab
4. Clip first end
5. Clip second end
6. Clip second end retaining claw
7. Housing
8. Outer body
8.1 Rib
9. Pin
10. Mouthpiece
11. Lid
12. Blister cavity
13. Blister
14. Guide gear recess
15. Guide gear
16. Inner body
17. Lower housing
18. Left center housing
19. Right center housing
20. Upper housing
21. Upper rolling gear of the blister lid
22. Intermediate rolling gear of the blister lid
23. Lower rolling gear of the blister lid
24. Trigger
25. Spring
26. Receptacle
27. Drive plate
28. Transmission gear
29. First additional gear
30. Second additional gear
31. Housing

### Detailed Description of the Invention

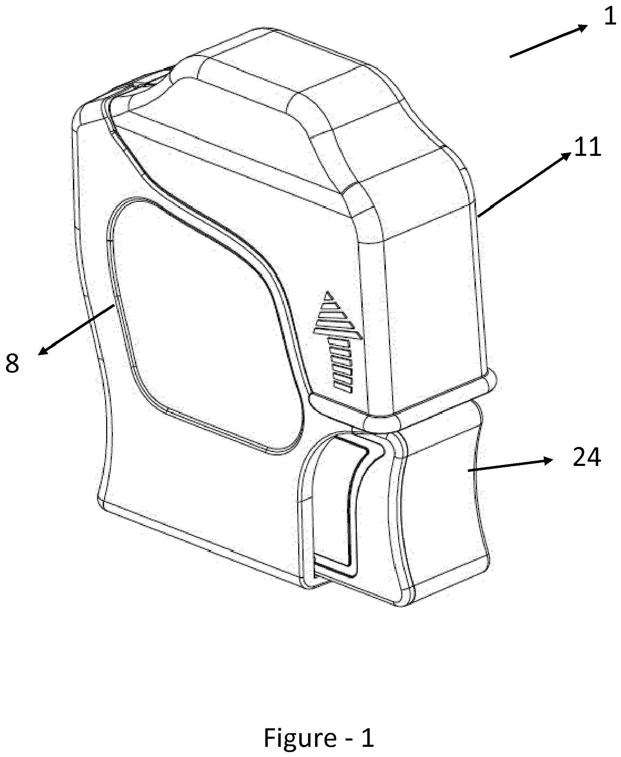

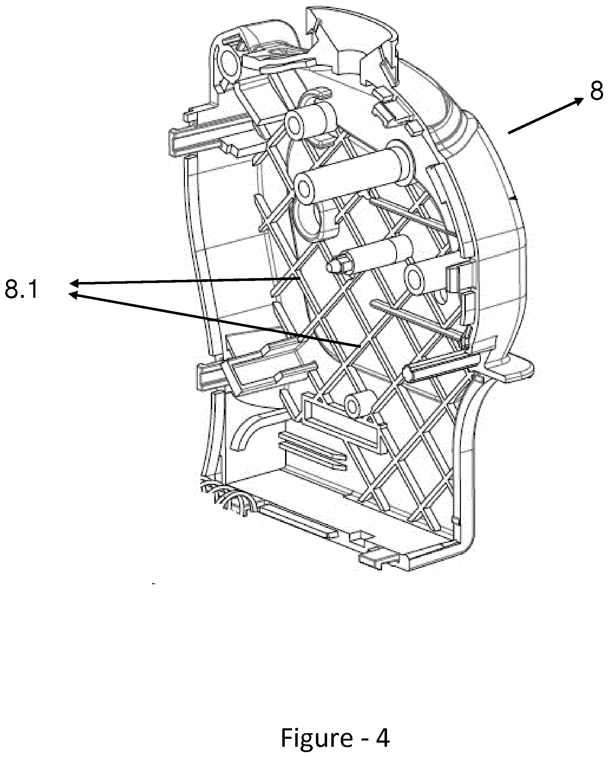

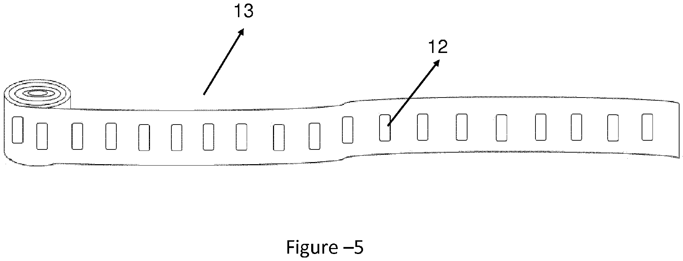

An outer body (8) of the inhaler device (1) of the present invention is explicated in an exemplary manner referring to the attached figures only so as to clarify without introducing any limiting effects.
An outer body (8) of the inhaler device (1) of the present invention of which the representative view is given in Figures 1, 2, 3, 4, 5 and 6 is composed of two compatible parts with retaining tabs disposed thereon. Ribs (8.1) extending in the horizontal and vertical directions are provided at the inner section of said parts so as to form a grid supporting the outer body (8). Said ribs (8.1) form parallel and intersecting structures along the body (8) and form a mesh-like, very strong structure protecting the inner surface area of the outer body (8) against factors such as weight, pressure, etc. Thus, the thickness of the polymer-based material forming the outer body (8) is significantly reduced with respect to the state of the art embodiments. The ribs (8.1) form grid structure. the grid form covers at least 50 % of the inner surface of the outer body (8). Consequently, advantage in volume and decrease in the amount of materials used are provided. Said rib (8.1) is shaped in one or more of the linear, S and Z forms. The S and Z-shaped rib (8.1) can be shaped in recurring Z and S forms. The rib (8.1) is preferably linear. Alternatively, said rib or ribs can be placed outer surface of the outer body (8).

Existing components are polymer-based, and produced and shaped by injection molding method.

The outer body (8) is formed by mounting said two parts to each other. A mouthpiece (10) enabling the user to inhale a medicament is provided at the upper part of said outer body (8). The mouthpiece (10) is composed of two parts, namely a section as an extension of the outer body (8) and an attachable/detachable headpiece. In order to keep the mouthpiece (10) clean and prevent foreign bodies from entering the inhaler device (1) when said inhaler device (1) is not being used, a lid (11) that pivots on the outer body (8) and that is closed on the mouthpiece (10) is provided.

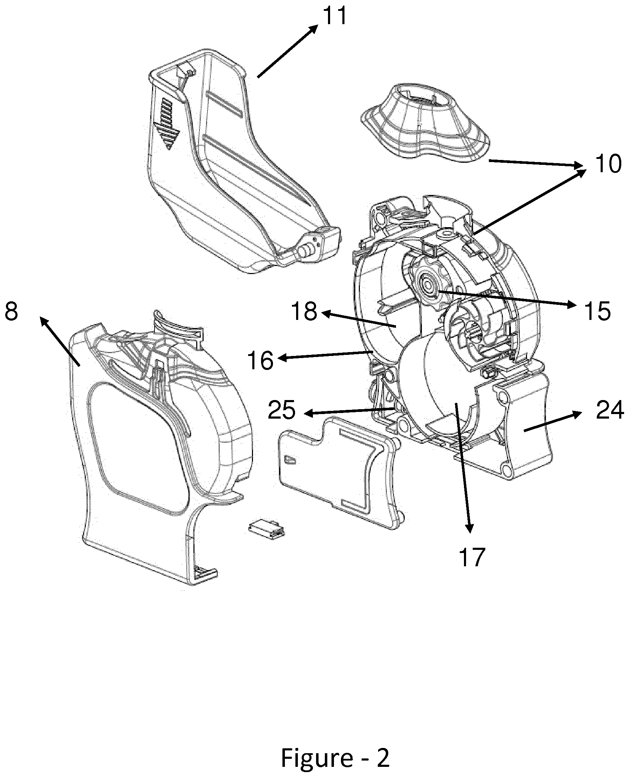

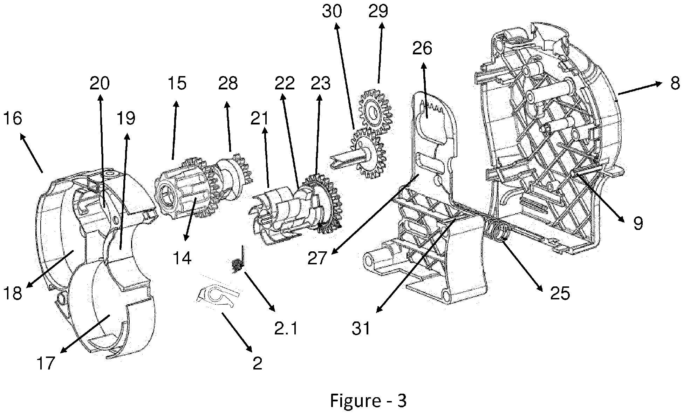

A blister (13) advancement mechanism gear group is disposed on the inside of the outer body (8) so as to provide the operation of the inhaler device (1). A blister (13) having cavities (12) filled with powder medicaments is positioned along the intermediate channels of said gear group. In said gear group, just under the mouthpiece (10), a guide gear (15) is provided, wherein the blister (13) cavities (12) filled with powder medicaments are disposed into recess (14) arranged on the body of the guide gear (15). The recesses (14) of the guide gear (15) are positioned right under the mouthpiece (10) of the device (1). An inner body (16) of which the representative view is given in Figures 2 and 3 is provided at the inner section of the outer body (8) so as to provide the positioning of the blister (13). The inner body (16) has a single-piece structure with all the lateral sides thereof being closed walls and allows the positioning of the blister (13) advancement mechanism gear group and of the blister (13). Said single-piece inner body (16) comprises a lower housing (17), a left center housing (18), a right center housing (19), an upper housing (20), recessed surfaces and tabs. The unused, full blister (13) is stored in the lower housing (17) and said strip-shaped blister (13) extends along the intermediate channels so as to be divided into two parts, namely the main layer and the lid layer, by means of the mechanism. The main layer of the blister (13) divided into two is rolled into the left center housing (18) and the lid layer thereof is rolled over the blister (13) lid upper rolling gear (21) that is positioned in the right center housing (19).

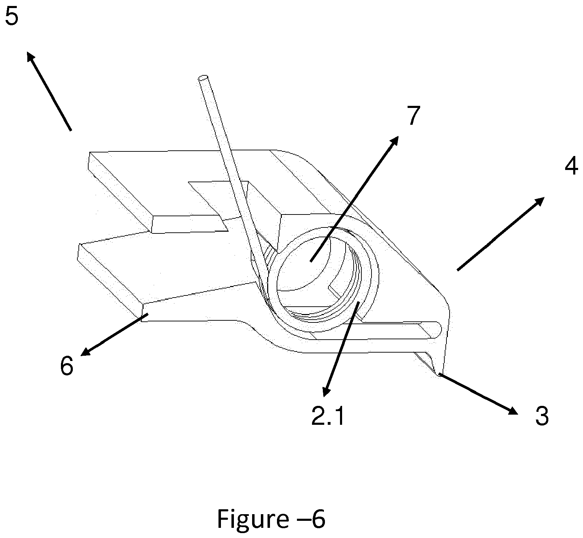

The clip (2) of which the representative view is given in Figure 6 is composed of a beak-shaped first end (4) having a retaining tab (3) and a second end (5) formed by two planar pieces. A second retaining tab (6) is provided at the extension of the second end (5). Moreover, a housing (7) is provided at the center of said second end (5). Said housing (7) in the clip (2) is connected to a pin (9) in the body (8) of the inhaler device (1). The connection between said housing (7) and the pin (9) enables said clip (2) to freely rotate. Said rotational movement is realized upwards and downwards in a limited manner. A spring (2.1) controls the limited rotation of the clip (2) and assures the stable position thereof in the horizontal axis. Said spring (2.1) is of helical type with one end, which constitutes the extension thereof, being linear. The helical section of the spring (2.1) is positioned in a housing formed in the body of the clip (2) while the linear section thereof is positioned in a housing formed at the inner edge surface of the outer body (8).

Thus, the linear section of said spring (2.1) is fixed in the housing at the inner edge surface of the outer body (8) so as to limit the rotational movement while the helical section in the clip (2) provides the free rotation.

At the beginning of the blister (13) advancement mechanism gear group of which the representative views are given in Figures 2, 3 and 4, a trigger (24) is provided, that is slidably disposed at the lower part of the outer body (8). Said trigger (24) can move in the axial/linear direction at the inner section of the outer body (8). The surface on the outer section of the trigger (24) on which the user applies pressure to push said trigger (24) inwards has a concave structure so as to provide ease of use. Between said trigger (24) and the inner surface of the outer body (8) a spring (25) is provided, that is compressed (loaded) when said trigger (24) is pushed into the outer body (8). Said spring (25) has a helical form.

In the inhaler device (1) of the present invention, a drive plate (27) is provided, that is fixed to said trigger (24) from one end and that has a receptacle (26) at the other end thereof. Said receptacle (26) is formed so as to be large enough to receive a gear therein. In the receptacle (26) of said drive plate (27), linear gears are provided, that are arranged in the axial/linear direction wherein the trigger (24) moves. The receptacle (26) is preferably rectangular and said gears are arranged at the upper edge of the receptacle (26). A transmission gear (28) is positioned in said receptacle (26). The transmission gear (28) is movably connected from the center thereof to a pin fixed to the inner body (16). Said connection allows the transmission gear (28) to make only rotational movement. A portion of the base peripheral surface of the transmission gear (28) contains teeth while the remaining portion does not have any teeth. The teeth of the transmission gear (28) engage with the linear gear of the drive plate (27). The surface of the transmission gear (28) comprising teeth in engagement with the linear gear realizes the rotation function. The surface portion without any teeth rotates inside the receptacle (26) without contacting any surfaces, thus providing an advantage in the volume and enabling the volume of the receptacle (26) to be in the acceptable range. Said transmission gear (28) engages with the guide gear (15). The guide gear (15) engages with the transmission gear (28) so as to identically imitate the movement of the transmission gear (28).

In light of the detailed description above, the inhaler device (1) of the present invention operates in the following manner. After the opening of the lid (11), the user applies force onto the gripping surface of the trigger (24). Afterwards the trigger (24) is slid into the outer body (8). With the axial/linear movement of said trigger (24), the drive plate (27) in connection with the trigger (24) is also slid. Thus, the transmission gear (28) in engagement with the linear gear of the drive plate (27) makes rotational movement. With the movement of the transmission gear (28), the guide gear (15) disposed thereon starts rotating and rotates the gears with which the guide gear (15) engages. Thus, the blister (13) positioned on the gears is moved and said blister (13) cavities (12) are opened.

The guide gear (15) of which the representative embodiment is shown in Figures 2 and 3 moves unidirectionally and realizes only the forward movement of the blister (13). The transmission gear (28) in engagement with the guide gear (15) can rotate forwards or backwards. By means of the guide gear (15), the transmission gear (28) transmits this movement to a first additional gear (29) and a second additional gear (30) as well as to the upper rolling gear (21), the intermediate rolling gear (22) and the lower rolling gear (23) of the blister (13) lid which engage with each other. While the transmission gear (28) and the guide gear (15) rotate counterclockwise, the lower rolling gear (23) of the blister (13) lid and the intermediate rolling gear (22) and the upper rolling gear (21) of the blister (13) lid which are positioned above the lower rolling gear (23) rotate clockwise. The blister (13) in engagement with said gears (21, 22, 23) moves through the channels of the inner body (16) and is stripped while passing through the blister (13) mechanism gear groups, thus the medicament in the blister (13) cavity (12) is exposed. The main layer of the blister (13) divided into two is rolled into the left center housing (18) and the lid layer thereof is rolled over the blister (13) lid upper rolling gear (21) that is positioned in the right center housing (19).

As a result of the trigger (24) being slid into the outer body (8), a housing (31) arranged on the trigger (24) fixedly engages with the locking clip (2) tab (3) positioned right thereabove, thus providing the use of a single dose of medicament. As the user continues to make the pushing movement until said locking position, the blister (13) is enabled to be completely stripped and the proper administration of required dose of medicament is assured. As a result of said locking, the trigger (24) is fixed and the pushing member is deactivated for a short period. Moreover, said pushing movement causes the spring (25) to be compressed between the inner surface of the outer body (8) and the trigger (24).

After the user closes the lid (11) following the use of the powder medicament, the lid (11) applies pressure onto the second end (5) of the locking clip (2) and lifts the first end (4), thus separating the tab (3) of the first end (4) of the clip (2) and the retaining housing (31) at the upper side of the trigger (24) from each other. Thus, the trigger (24) reaches to the exact pressing position. A very low spring (25) force is required to slide the trigger (24) backwards so as to make the device (1) ready for use.

As the trigger (24) is slid backwards, the mechanism rotates the transmission gear (28) in the reverse direction, thus providing the reverse movement of the mechanism. Thus, the device (1) becomes ready for reuse without the need for the user to wind up the same. However, the mechanism cannot move the blister (13) in the reverse direction. In light of the description given herein, it is clear that the inhaler device (1) of the present invention can be used only with a single pushing movement by the user. With the closing of the lid (11), the device (1) is automatically wound up and the device (1) becomes ready for reuse.

The linear movement of the trigger (24) is realized as the trigger (24) being slid in any direction including the x and y axes. Said direction is determined according to the design of the device (1).

The term "blister (13) advancement mechanism gear group" comprises the guide gear (15), the transmission gear (28), the blister (13) lid lower rolling gear (23), the blister (13) lid intermediate rolling gear (22), the blister (13) lid upper rolling gear (21), the first additional gear (29) and the second additional gear (30), and other wheels and gears that can be added to the main components of the device (1) are considered within the scope of this term.

Consequently, by means of the described embodiment, an inhaler device (1) having an outer body (8) resistant to outer factors and operating with precision and stability is realized.

## Claims

1. An dry powder inhaler device (1) comprising an outer body (8)
- **characterized in that** said outer body (8) comprises at least one rib (8.1) at the inner surface thereof.

2. An dry powder inhaler device (1) as in Claim 1, wherein said rib (8.1) is shaped in one or more of the linear, S and Z forms.

3. An dry powder inhaler device (1) as in Claim 1 or 2, wherein the rib (8.1) is preferably linear.

4. An dry powder inhaler device (1) as in any one of the above claims wherein two or more ribs (8.1) are provided.

5. An dry powder inhaler device (1) as in any one of the above claims, wherein said ribs (8.1) are positioned parallel to each other.

6. An dry powder inhaler device (1) as in any one of the above claims, wherein the ribs (8.1) are positioned so as to intersect each other.

7. An dry powder inhaler device (1) as in any one of the above claims, wherein the ribs (8.1) are in a grid form.

8. An dry powder inhaler device (1) as in any one of the above claims, wherein the grid form covers at least 50 % of the inner surface of the outer body (8).
